# EUROPEAN PATENT SPECIFICATION

(11) **EP 0 825 869 B1**
(45) Date of publication and mention of the grant of the patent: **07.08.2002**
(21) Application number: 96914505.1
(22) Date of filing: 08.05.1996
(51) Int. Cl.: A61K 35/74, A61K 31/195, A61P 1/16

(54) **PHARMACEUTICAL COMPOSITION COMPRISING LACTOBACILLUS PLANTARUM AND ARGININE**
PHARMAZEUTISCHE ZUSAMMENSETZUNGEN WELCHE LACTOBACILLUS PLANTARUM UND ARGININ BEINHALTEN
COMPOSITION PHARMACEUTIQUE A BASE DE LACTOBACILLUS PLANTARUM ET D'ARGININE

(30) Priority: 09.05.1995 SE 9501719
(43) Date of publication of application: 04.03.1998
(73) Proprietor: PROBI AB, 223 70 Lund (SE)
(72) Inventor: ADAWI, Diya, S-226 46 Lund (SE); JEPPSON, Bengt, S-226 47 Lund (SE); MOLIN, Göran, S-224 67 Lund (SE)
(74) Representative: Larfeldt, Helene
(86) International application number: SE9600603
(87) International publication number: WO9635440

(56) References cited:
- DIALOG INFORMATION SERVICES, File 350, Dialog Accession No. 000531012, WPI Accession No. 66-31586/F/00, AJINOMOTO CO LTD, "Lactic Acid Bacteria Prepns. Contng. Amino Acids as Stabilisers"; & JP,B,43 008 009, 6800, (BASIC).
- ANN. INST. PASTEUR/MICROBIOL., Volume 137A, 1986, E.N. MOYEN et al., "Modification Par L'Erythromycine et un Extrait de Lactobacillus Acidophilus de La Colonisation De L'Instestin et de La Translocation de Campylobacter Jejuni Chez La Souris Axenique", pages 199-207.
- ANNALS OF SURGERY, Volume 217, No. 6, 1993, LUCA GIANOTTI et al., "Arginine-Supplemented Diets Improve Survival in Gut-Derived Sepsis and Peritonitis by Modulating Bacterial Clearance", pages 644-653.
- AACN CLINICAL ISSUES, Volume 5, No. 4, 1994, LINDA M. LORD et al., "The Role of the Gut in Critical Illness", pages 450-458.
- DIALOG INFORMATION SERVICES, File 155, Medline, Dialog Accession No. 08912145, Medline Accession No. 94227145, DE OCA J., "Bacterial Translocation from the Nutritional Perspective la Translocation Bacteriana Desde la Perspectiva Nutricional"; & NUTR. HOSP. (SPAIN), Jan-Feb. 1994, 9(1), p2-11.

## Description

The present invention refers to a pharmaceutical composition comprising a Lactobacillus plantarum strain having the ability to colonize or adhere to the human intestinal mucosa in combination with arginine. Said composition can be used for the prophylactic or curative treatment of acute liver injury and failure as well as associated conditions in which there is a defective mucosal membrane barrier and increased bacterial translocation.

### Background of the invention

Bacterial translocation, that is the escape of microorganisms and toxic compounds from the gut lumen by crossing the intestinal mucosal barrier or by traversing the bowel wall, is of essential importance in the development of many disorders. Bacterial translocation can for instance occur in immunocompromised patients, in critically ill and multiple organ failure patients, multiple trauma patients and burn cases, in liver diseases and inflammatory bowel diseases, and in patients suffering from malnutrition. Viral hepatitis, fulminant hepatic failure, cirrhosis, alcoholic hepatitis, cholangitis and autoimmune diseases such as chronic active hepatitis are liver diseases in which bacterial translocation can take place.

In acute liver failure following hepatitis, toxic insults or after major liver surgery, there is an increased bacterial translocation from the gut. This may explain some of the infectious complications seen in these conditions. Acute liver failure has a high mortality rate and a significant proportion of the mortality can be attributed to the high incidence of sepsis. In critically ill or immunocompromised patients most infections are caused by the patients own microflora and many patients dying of sepsis or multiple system organ failure have enteric bacteremi for which no septic focus is identified, indicating that these infections may have originated from the gut. Because of the high frequency of clinically significant bacterial sepsis during fulminant hepatic failure there is also a need of prophylactic treatment.

Bacterial translocation can lead to sepsis and septisemia, which potentiate the liver failure and can lead to multiple organ failure. The treatment of the increased bacterial infection is by using antibiotics, which has many side effects and can affect the intestinal mucosal barrier and microflora.

These findings have led to an increased interest for microbial species which can beneficially affect the microbial balance of the host, e.g. by producing antimicrobial components or by competitive growing. Lactobacillus has been among the most studied species, and have in certain instances been shown to counteract the proliferation of pathogens. Lactobacilli constitute an integral part of the normal gastrointestinal microeco-logy and are involved in host metabolism. Bacteriotherapy with lactobacilli has been reported to be effective in both pseudomembraneous and ulcerative colitis.

The amino acid arginine has a potent effect on body immune system, particularly after trauma. Furthermore arginine is a known precursor of polyamines which are considered important mediators of cell growth and differentiation. It is also known that arginine stimulates the local and systemic immune system.

### Prior art

SE 9501056-7 refers to the use of certain Lactobacillus strains having an adhesin conferring adherence to a receptor in human intestinal epithelial cells for the preparation of a pharmaceutical composition triggering the immune response, and also for treatment of translocation of pathogenic or potentially pathogenic bacteria over intact intestinal epithelium. Said application especially refers to the use of Lactobacillus plantarum 299, DSM 6595, and Lactobacillus plantarum 299v, DSM 9843, as well as other Lactobacillus plantarum strains belonging to a cluster as specified.

Gianotti, Luca et al., Annals of surgery, Vol 217, No. 6, 644-654, investigate the effect of dietary arginine on bacterial translocation. An increased survival could be observed in animals fed with an arginine-supplemented diet. Quantitative colony counts and the calculated percentage of remaining viable bacteria showed that the ability to kill translocated organisms was significantly enhanced in animals receiving arginine.

Daly, John E., et al., Surgery 112:55-67, 1992 show that enteral nutrition with supplemental arginine, RNA and omega-3-fatty acids in patients after operation improves the immune defence through different mechanisms.

### Description of the invention

It has now surprisingly been found that a combination of certain strains of Lactobacillus and arginine significantly reduces the degree of liver injury and failure associated with bacterial translocation.

The invention refers to a pharmaceutical composition comprising
- a Lactobacillus plantarum strain having the ability to colonize human intestinal mucosa in vivo;
- arginine; and
- a pharmaceutically acceptable carrier.

The invention especially refers to a pharmaceutical composition wherein the Lactobacillus strain has an adhesin conferring adherence to a receptor on human intestinal epithelial cells. The adhesion between the glycoprotein receptor and the adhesin is inhibited by the sugar α-methyl mannoside, which implies that the receptor is mannose-containing. This receptor is, however, different from the mannose-containing receptor for E. coli type 1 fimbriae, which can be shown by means of hemagglutination tests. It is believed that the receptor contains the Manα1-2Man sequence.

The invention especially refers to a pharmaceutical composition wherein the Lactobacillus plantarum strain belongs to a cluster having a restriction endonuclease analysis similarity of more than 70 % to strain Lactobacillus plantarum 299, deposition number DSM 6595, by using the Pearson product moment correlation coefficient and the unweighed pair group algorithm with aritmethic averages (UPGMA; GelCompare 3.0, Applied Maths, Kortrijk, Belgium).

Restriction endonuclease analysis, REA, refers to analysis of the cleavage pattern formed in electrophoresis on agar gel of the bacterial chromosomal DNA which has been decomposed with restriction enzymes according to the method described below under Genotype identification. By characterization of the strains by means of their REA-pattern the identity of the used isolates can be established.

In particular the invention refers to a composition comprising any of the strains

| | |
|---|---|
| Lactobacillus plantarum 299 | DSM 6595 |
| Lactobacillus plantarum 299v | DSM 9843 |
| Lactobacillus plantarum 79 | |
| Lactobacillus plantarum 105 | |
| Lactobacillus plantarum 107 | |

or other strains with more than 70 % similarity to L. plantarum 299 in terms of REA.

The preferred Lactobacillus plantarum strains are described in our copending application SE 9501056-7.

Arginine in the composition preferably is L-arginine. It is however also possible to use a protein rich in arginine, such as cottonseed protein, or a protein from soybeens or peas.

The pharmaceutical composition of the invention comprises in addition to the Lactobacillus strain and arginine also a carrier which is pharmaceutically acceptable. Conventional carriers are for example physiologically acceptable substrates fermented by the bacterium in question, as well as foodstuffs of various kinds, especially based on starch or milk, but also inert solid or liquid substances, such as saline or water. A suitable substrate should contain liquid or solid fibres which are not resorbed in the gastro-intestinal tract and which when fermented with Lactobacillus form short fatty acids. As an example of suitable, starchcontaining substrates can be mentioned cereals, such as oats and wheat, corn, root vegetables such as potatoes and certain fruits such as green bananas.

The composition according to the invention is administrable in any suitable way, preferably orally or rectally, for example in the form of enema. It is also administrable enterally through a catheter inserted in the intestines via the stomach or directly in the intestines. Tests have shown that the effect is improved if dietary fibres in the form of for example oatmeal gruel or of β-glucans are supplied. The treatment should take place once or several times daily for a period of 1 - 2 weeks.

In liver failure, for instance in connection with liver surgery and liver inflammation, the liver does not fulfill its normal functions, but ammonia, nitrogen compounds, endotoxins and other bacterial products will pass through the liver without being metabolized and effect the whole body. One way to reduce the load on the liver during these circumstances is to give the patient an enema or to provoke a diarrhea in order to rinse the intestines. In doing this many products which are needed for the intestines to function are removed. A composition of the invention preferably is administrable as an enema in a situation like this.

The Lactobacillus strain can preferably be used in a concentration of 10⁷-10⁹ CFU/ml in the pharmaceutical composition. The preferred dose of arginine to a human is 25-30 g/d.

A preferred carrier for oral administration is a lactic acid fermented food product, such as a nutrient solution based on oatmeal, for instance as described in WO 89/08405, or based on milk, for instance cheese. Another preferred carrier is a diet fibre product, such as beta-glucans, pectin, oligosaccharides, which in turn can be included in the lactic acid fermented food product. In oral administration the preferred amount to be given, 1-5 times a day, is 100-200 ml.

A preferred composition for oral administration comprises the arginine component protected from being digested in the small intestine, for instance by means of preaggregation of arginine with a gelforming polysaccharide which, when swelling in the gastrointestinal tract will provide a protective gel coating up to the fermentation in the colon. This type of gel coating can be performed with pectins, guar seed powder and glucomannane from the Konjac plant, such as the commercial product Propal (Shimizu Co., Japan).

In rectal administration the carrier is preferably a physiological solution in which the arginine and Lactobacillus are suspended, optionally together with a diet fibre as beta-glucan or pectin. The preferred amount given, 1-2 times a day, is then 20-50 ml.

### Brief description of the drawings

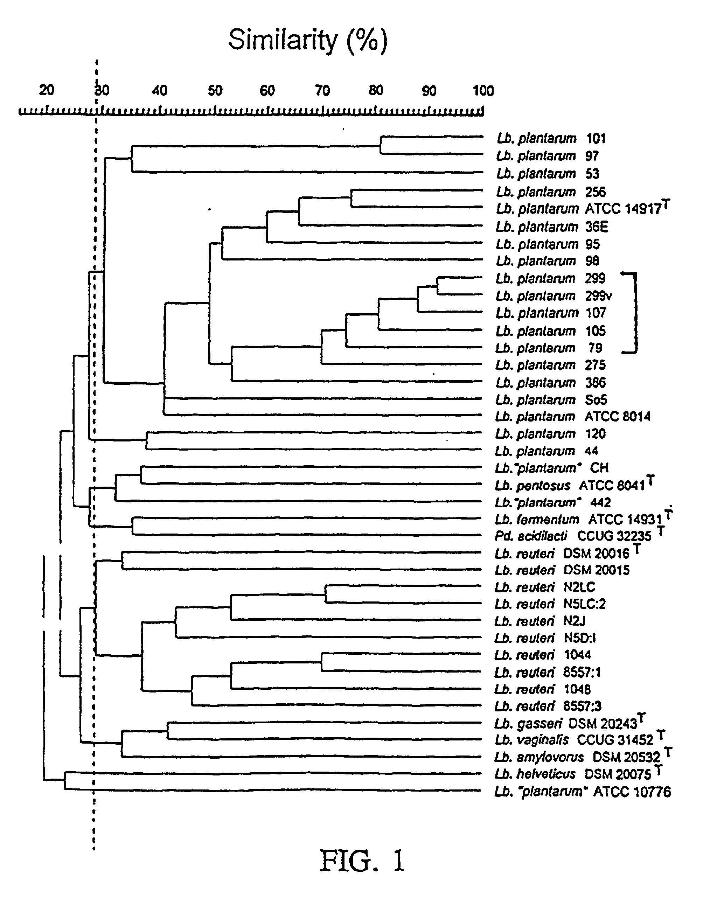

Figure 1 is a dendrogram showing the similarity in % between different tested strains of Lactobacillus which have been characterized by the REA-method, based on the Pearson product moment correlation coefficient and UPGMA.

### Identification of strains

The strains 299 and 299v, which were both isolated from healthy human intestinal mucosa, have been deposited at the Deutsche Sammlung von Mikroorganismen und Zellkulturen GmbH on July 2, 1991 and March 16, 1995, respectively, and have been given the deposition numbers DSM 6595 (299) and DSM 9843 (299v).

Phenotypic identification The strains 299, 299v, 79, 105 and 107 are Gram positive, catalase-negative rods growing on Rogosa agar at pH 5.5 and capable of producing lactic acid from glucose anaerobically. The ability of the strains to ferment different carbohydrates is shown in Table 1. The tests have been carried out by means of the API 50 CH in accordance with the instructions of the manufacturer.

**Table 1**

| Fermentation patterns of strains L. plantarum 299, L. plantarum 299v, L. plantarum 107, L. plantarum 105 and L. plantarum 275 on API 50CH at 30°C and 37°C. | | | | | | | | | | |
|---|---|---|---|---|---|---|---|---|---|---|
| *carbohydrate* | *L.plantarum* | | *L.plantarum* | | *L.plantarum* | | *L.plantarum* | | *L.plantarum* | |
| | *299* | | *299v* | | *107* | | *105* | | | *79* |
| | *30°C* | *37°C* | *30°C* | *37°C* | *30°C* | *37°C* | *30°C* | *37°C* | *30°C* | *37°C* |
| Glycerol | - | - | - | - | - | - | - | - | - | - |
| Erythritol | - | - | - | - | - | - | - | - | - | - |
| D-arabinose | - | - | - | - | - | - | - | - | | -- |
| L-arabinose | + | + | + | + | + | + | + | + | | ++ |
| Ribose | + | + | + | + | + | + | + | + | + | + |
| D-xylose | - | - | - | - | - | - | - | - | | -- |
| L-xylose | - | - | - | - | - | - | - | - | - | - |
| Adonitol | - | - | - | - | - | - | - | - | - | - |
| β-methylxyloside | - | - | - | - | - | - | - | - | - | - |
| Galactose | + | + | + | + | + | + | + | + | + | + |
| D-glucose | + | + | + | + | + | + | + | + | + | + |
| D-fructose | + | + | + | + | + | + | + | + | + | + |
| D-mannose | + | + | + | + | + | + | + | + | + | + |
| L-sorbose | - | - | - | - | - | - | - | - | - | -- |
| Rhamnose | - | - | - | - | - | - | - | - | - | - |
| Dulcitol | - | - | - | - | - | - | - | - | - | - |
| Inositol | - | - | - | - | - | - | - | - | - | - |
| Mannitol | + | + | + | + | + | + | + | + | + | + |
| Sorbitol | + | + | + | + | + | + | + | + | + | + |
| α-methyl-D-mannoside | + | + | + | + | + | + | + | + | | ++ |
| α-methyl-D-glucoside | - | - | - | - | | - | - | - | - | -- |
| N-acetylglucoseamine | + | + | + | + | + | + | + | + | + | + |
| Amygdaline | + | + | + | + | + | + | + | + | + | + |
| Arbutin | + | + | + | + | + | + | + | + | + | + |
| Aesculin | + | + | + | + | + | + | + | + | + | + |
| Salicine | + | + | + | + | + | + | + | + | + | + |
| Cellobiose | + | + | + | + | + | + | + | + | + | + |
| Maltose | + | + | + | + | + | + | + | + | + | + |
| Lactose | + | + | + | + | + | + | + | + | + | + |
| Melibiose | + | + | + | + | + | + | + | + | + | + |
| Saccharose | + | + | + | + | + | + | + | + | + | + |
| Trehalose | + | + | + | + | + | + | + | + | + | + |
| Inulin | - | - | - | - | - | - | - | - | - | - |
| Melezitose | + | + | + | + | + | + | + | + | + | + |
| D-raffinose | - | - | + | - | + | - | - | - | - | - |
| Starch | - | - | - | - | - | - | - | - | - | - |
| Glycogen | - | - | - | - | - | - | - | - | - | - |
| Xylitol | - | - | - | - | - | - | - | - | - | - |
| β-gentiobiose | + | + | + | + | + | + | + | + | + | + |
| D-turanose | + | + | + | + | + | + | + | + | + | + |
| D-lyxose | - | - | - | - | - | - | - | - | - | - |
| D-tagarose | - | - | - | - | - | | - | - | - | - |
| D-fucose | - | - | - | - | - | - | - | - | - | - |
| L-fucose | - | - | - | - | - | - | - | - | - | - |
| D-arabitol | - | - | + | - | + | - | + | - | + | - |
| L-arabitol | - | - | - | - | - | - | - | - | - | - |
| Gluconate | + | + | + | + | + | + | + | + | + | + |
| 2-cetogluconate | - | - | - | - | - | - | - | - | - | - |
| 5-cetogluconate | - | - | - | - | | - | - | - | - | - |

Phenotypically the strains can be identified as Lactobacillus plantarum.

### Genotypic identification

### REA

The strains have been examined as to the cleavage pattern of the chromosomal DNA, through restriction-endonuclease analysis - REA - method according to Ståhl M, Molin G, Persson A, Ahrné S & Ståhl S, International Journal of Systematic Bacteriology, 40:189-193, 1990, and further developed by Johansson, M-L, et al., International Journal of Systematic Bacteriology 45:670-675, 1995. Schematically REA can be described as follows: Chromosomal DNA from the strains involved in the study were prepared and cleaved by restriction endonucleases. 0.75 µg of each DNA was separately digested at 37°C for 4 h with 10 units of HindIII, ClaI and EcoRI; each endonuclease was used separately. Ståhl et al, above, used Asp718 instead of Hind III, but digestion with Asp718 resulted in too many large bands to be optimal for the present methodology. The cleaved DNA fragments are separated as to size by gel electrophoresis using submerged horizontal agarose slab gels. The gels consisted of 150 ml of 0.9 % agaroses (ultrapure DNA grade; low electro-endo osmosis; BioRad Laboratories, Richmond, USA) and were cast as slab gels (150 by 235 mm). 0.2 µg of a High Molecular Weight DNA marker (Bethesda Research Laboratories, BRL) together with 0.5 µg of a DNA molecular weight marker VI (Boehringer Mannheim, Germany) were used as standards. Additionally pHC 79 digested with EcoRI (molecular weight 4.2 x 10⁶; Boehringer Mannheim) was added to each well as an internal standard. Minimal band distortion and maximal sharpness were achieved by applying the sample DNA in Ficoll loading buffer (2g of Ficoll, 8 ml of water, 0.25% bromphenol).

Gels were run at a constant voltage of 40V for 18h at 8.5°C. The buffer (89 mM Tris, 23 mM H₃PO₄, 2 mM sodium EDTA, pH 8.3) was recirculated during the running period. Thereafter, the gels were stained for 20 minutes in ethidium bromide (2 µg/ml) and destained in distilled water, visualized at 302 nm with a UV transilluminator (UVP Inc., San Gabriel, USA) and photographed. This way of running the gel electrophoresis gave well distributed and relatively well-separated band down to a molecular weight of 1.2 x 10⁶.

Band patterns on photo negatives were scanned with a laser densitometer (UltroScan XL; LKB-Produkter AB, Bromma, Sweden). The resolution was 1000 points per lane, and each lane was about 50 mm long. Data were obtained by using the LKB 2400 GelScan XL software package (LKB-Produkter AB). The band patterns were normalized and background noise was subtracted using the rolling-disc algorithm of the GelCompar 3.0 program (Applied Maths, Kortrijk, Belgium). The data from the three cleavages (by HindIII, ClaI and EcoRI) were combined for each strain (using Gel Compar 3.0).

The data set was analyzed by using the Pearson product moment correlation coefficient (r) and the unweighed pair group algorithm with arithmetic averages (UPGMA) (Romersburg, H.C. 1984, Cluster analysis for research. Lifetime Learning Publications, Belmont, California, USA). The Pearson correlation coefficients and the UPGMA values were calculated for the complete data set and the GelCompar 3.0 program was used. In Fig. 1 the cluster having a similarity >70 % to L. plantarum 299 has been marked out.

### Plasmid profiling

The strains were tested according to the method described by Chassy et al (1976) as to the contents of plasmids. Lactobacillus plantarum 299, 299v, 79 and 105 had identical plasmid profiles, i.e. five plasmids of 4, 9, 15, 21 and >30 MDa. Lactobacillus plantarum 107 had three plasmids of 4, 15 and 21 MDa.

### Biological tests in rats

The aim of this experiment is to study the effect of rectal supplementation of different strains of Lactobacillus with and without arginine on the extent of liver injury and bacterial translocation in an acute liver injury model.

Five different Lactobacillus strains were used in the experiment: L. reuteri R2LC isolated from rat intestinal mucosa, L. rhamnosus DSM 6594 (strain 271) isolated from human colon, L. fermentum 8704:3 (strain 245) isolated from human jejunum, L. reuteri 108 isolated from human jejunum and L. plantarum DSM 9843 (strain 299v) isolated from human jejunum and rectum. All bacterial strains were obtained from the Laboratory of Food Hygiene, Dept. of Food Technology, Lund University, Lund, Sweden.

Male Sprague-Dawley rats with a weight range of 200-300 g were divided into 13 groups of six animals: normal, control acute liver injury (ALI), supplemented arginine (SA), supplemented Lactobacillus reuteri R2CL (SR), supplemented Lactobacillus reuteri R2CL + arginine (SRA), supplemented Lactobacillus rhamnosus 271 (SS), supplemented Lactobacillus rhamnosus 271 + arginine (SSA), supplemented Lactobacillus plantarum 299v (SP), supplemented Lactobacillus plantarum 299v + arginine (SPA), supplemented Lactobacillus fermentum 8704:3 (SF), supplemented Lactobacillus fermentum 8704:3 + arginine (SFA), supplemented Lactobacillus reuteri 108 (ST) and supplemented Lactobacillus reuteri 108 + arginine (STA). All animals received normal rat chow (R3, Lactamin AB, Stockholm) and water ad libitum throughout the experiment and were kept at 12 hours light/dark cycle and 22°C room temperature. The different Lactobacillus strains were administered rectally once daily for 8 days with and without 2 % arginine through a rectal tube. The daily supplementation of the Lactobacillus strains was about 3x10⁹ CFU per animal in 3 ml. Acute liver injury was induced on the 8th day by intraperitoneal injection of D-galactoseamine (Sigma Chemical Co., St Louis, USA), 1.1 g/kg body weight, which brings about an increased leakage of bacteria from the intestinal lumen to distant organs. In the acute liver control group, normal saline was supplemented daily for 8 days and the liver injury induced on the 8th day. Samples were collected 24 and 48 hours after the evocation of the liver injury. Under ether anesthesia a laparotomy was performed through a midline incision under aseptic technique. Portal blood was collected for bacteriological tests and arterial blood was collected for bacteriological and liver enzymes tests. Samples from the caudate lobe of the liver and mesenteric lymph nodes (MLN) were obtained for bacteriological and liver histology studies.

Incidence of translocated bacteria. In the bacteriological analysis blood samples were immediately placed in EDTA containing sterile tubes. Tissue samples were placed in 5 ml of sterile transport medium. The samples were placed in ultrasonic bath for 5 minutes and swirled on Chiltern (Terma-Glas, Sweden) for 2 minutes. Total aerobic plate count was made by placing 1.0 ml of the sample on brain heart infusion agar BHI (Oxoid) and incubated at 37°C for 3 days. Total anaerobic plate count was made by placing the samples on BHI and incubating under anaerobic condition at 37°C. After 3 days the number of colonies formed on each plate were counted and corrected for the weight of the original tissue. The incidence of bacterial translocation in different parts of each rat of the respective group of six rats

**Table 1**

| Incidence of bacterial translocation after 24 h | | | | |
|---|---|---|---|---|
| Group | Portal blood | Arterial blood | Liver | MLN |
| Normal | 0/6 | 0/6 | 0/6 | 0/6 |
| ALI | 5/6 | 6/6 | 6/6 | 6/6 |
| SA | 5/6 | 3/6 | 6/6 | 4/6 |
| SRA | 1/6* | 1/6** | 4/6 | 2/6* |
| SR | 2/6 | 1/6** | 5/6 | 3/6 |
| SSA | 5/6 | 4/6 | 6/6 | 6/6 |
| SS | 3/6 | 3/6 | 6/6 | 6/6 |
| SPA | 2/6 | 1/6** | 5/6 | 5/6 |
| SP | 3/6 | 2/6* | 4/6 | 2/6* |
| SFA | 1/6* | 2/6* | 5/6 | 5/6 |
| SF | 5/6 | 2/6* | 5/6 | 5/6 |
| STA | 1/6* | 1/6** | 4/6 | 4/6 |
| ST | 2/6 | 1/6** | 4/6 | 4/6 |

| | | | | |
|---|---|---|---|---|
| * denotes p < 0.05, | | | | |
| ** denotes p < 0.01 in comparison to the acute liver control group | | | | |

In the scoring of the incidence of bacterial translocation above the presence or absence of bacteria in the blood or tissue of the rat is stated; that is as a positive value is given to a rat if at least one bacterium is found. The incidence of bacterial translocation was evaluated using Fisher's exact test. A probability level less than 0.05 was considered to be significant.

As can be seen from Table 1 the incidence of bacterial translocation to the blood increased significantly in the acute liver injury control group compared to the normal control. The incidence of bacterial translocation was, however, significantly reduced in the groups supplemented with L. plantarum 299v, L. reuteri R2LC and 108, and L. fermentum 8704:3 with and without arginine. The incidence of translocated bacteria in the liver and mesenteric lymph nodes decreased slightly in all groups compared to the acute liver injury control group.

Liver enzymes in the blood. The liver enzymes serum bilirubin (Bil), alkaline phosphatase (ALP), aspartate aminotransferase (ASAT) and alanine aminotransferase (ALAT) levels in arterial blood were measured after centrifugation of the blood (1000xg, 10 min) on a Kodak, Echa chem 700 XRC, in accordance to the recommendations of the Committee on the Enzymes of the Scandinavian Society for Clinical Chemistry and Clinical Physiology (Scan J Clin Lab Inves 1974; 33:291-306). The serum amino-transferases are sensitive indicators of liver cell injury and most helpful in recognizing acute hepatocellular diseases and they are the most frequently measured indicators of liver diseases. Hepatobiliary diseases lead to the elevation of serum alkaline phosphatase. In hepatocellular damage the serum bilirubin values are higher. The combination of an increased serum bilirubin, an elevated ASAT or ALAT, and an elevated alkaline phosphatase has a predictive accuracy for the presence of liver dieases.

All values are presented as mean ± SEM. The results have been statistically evaluated using all pairwise multiple comparison methods.

**Table 2**

| Level of liver enzymes in the arterial blood | | | | |
|---|---|---|---|---|
| Groups | ALP (µKat/l) | Bil (µmol/l) | ASAT (µKat/l) | ALAT (µKat/l) |
| ALI | 16.43±1.41 | 16,17±3,36 | 97.83±15.90 | 86.17±16.44 |
| SA | 14,86±1.12 | 14.57±2.20 | 59.83±12.54 | 50.50±8.89 |
| SRA | 14.55±1.48 | 14.50±4.15 | 60.42±12.92 | 48.87±12.04 |
| SR | 14.83±0.60 | 15.00±1.46 | 62.67±3.76 | 58.83±9.64 |
| SSA | 15.60±1.69 | 15.80±4.72 | 65.17±21.13 | 60.50±17.99 |
| SS | 16.20±2.58 | 15.62±4.20 | 65.57±23.35 | 64.03±13.97 |
| SPA | 12.83±0.83 | 6.58±1.56 | 23.00±4.57* | 25.32±5.16 |
| SP | 13.67±1.38 | 10.85±2.40 | 45.18±10.23 | 44.67±10.84 |
| SFA | 14.67±1.43 | 13.83±2.98 | 55.50±16.67 | 56.55±17.03 |
| SF | 14.92±0.84 | 14.83±5.69 | 57.35±13.53 | 57.67±13.20 |
| STA | 13.67±1.33 | 12.00±5.14 | 46.17±11.63 | 41.83±10.68 |
| ST | 14.17±1.19 | 14.00±1.98 | 59.67±12.93 | 58.33±11.57 |

| | | | | |
|---|---|---|---|---|
| * p < 0.05 compared to the ALI group using Neuman-Keuls all pairwise multiple comparison method | | | | |

From the above table can be seen that the liver enzymes decreased in all the groups supplemented with Lactobacillus with and without arginine, and that said decrease was most significant in the group supplemented with the combination of arginine and Lactobacillus plantarum.

Liver histology. Samples from the lobe of the liver were placed in 4 % phosphate buffered formaldehyde. Paraffin-embedded samples were sliced and studied under light microscopy after staining with hematoxylin and eosin. At least three slides were studied from each specimen. Liver histological scoring was done according to Chojkier, M. et al., Gastroenterology 1985; 88:115-21 and Shiratori, Y. et al., Hepatology 1988;8(4): 815-21 with slight modification. The degree av hepatocellular necrosis and inflammatory cell infiltration was quantitatively assessed blindly at the Department of Pathology at Lund University as follows:
1 minimal
2 moderate
3 submassive
4 massive
The results of the test are given in Table 3 below.

**Table 3**

| Total histological score of the liver | | | |
|---|---|---|---|
| 24 hours | | 48 hours | |
| Groups | Total score | Groups | Total Score |
| ALI | 4.8±0.3 | ALI | 5.3±0.5 |
| SA | 4.3±0.2 | SA | 3.3±0.2* |
| SRA | 3.5±0.6 | SPA | 3.0±0.6* |
| SR | 4.0±0.3 | SP | 3.7±0.2 |
| SSA | 4.3±0.2 | | |
| SS | 4.2±0.4 | | |
| SPA | 2.8±0.2* | | |
| SP | 4.0±0.0 | | |
| SFA | 4.2±0.5 | | |
| SF | 4.0±0.5 | | |
| STA | 4.0±0.0 | | |
| ST | 4.3±0.3 | | |

| | | | |
|---|---|---|---|
| * p < 0.05 compared to the ALI group using Dunn's all pairwise multiple comparison method | | | |

Twenty four hours, as well as 48 hours, after the liver injury, the liver histological score showed a significant difference in the supplemented L. plantarum + arginine group compared to the acute liver injury control group.

### Summary

The incidence of infections with intestinal bacteria in patients with acute liver failure of different etiology is very high, about 30-40 %. Translocation of the bacteria to the extraintestinal sites potentiate the liver failure and can lead to septicemia and multiple organ failure. From the above tests can be concluded that a composition of the invention comprising arginine and Lactobacillus plantarum strains seemed to be superior to the other tested strains in preventing liver injury and failure and associated bacterial translocation, as can be judged by the significant decrease in the release of liver enzymes and bilirubin, decrease in bacterial translocation as well as from the liver histology scores.

## Claims

1. A pharmaceutical composition comprising
- a Lactobacillus plantarum strain having the ability to colonize human intestinal mucosa in vivo;
- arginine; and
- a pharmaceutically acceptable carrier.

2. A pharmaceutical composition according to claim 1, wherein the Lactobacillus plantarum strain has an adhesin conferring adherence to a receptor on human intestinal epithelial cells.

3. A pharmaceutical composition according to claim 1 or 2, wherein the Lactobacillus strain is a strain of Lactobacillus plantarum belonging to a cluster having a restriction endonuclease analysis similarity of more than 70 % to a strain Lactobacillus plantarum 299, deposition number DSM 6595, by usin the Pearson product moment correlation coefficient and the unweighed pair group algorithm with aritmethic averages (UPGMA; GelCompare 3.0, Applied Maths, Kortrijk, Belgium).

4. A pharmaceutical composition according to any of claims 1-3, wherein the Lactobacillus plantarum strain is selected from the group consisting of Lactobacillus plantarum 299, DSM 6595, and Lactobacillus plantarum 299v, DSM 9843.

5. A pharmaceutical composition according to any of claims 1-4, wherein the arginine is L-arginine.

6. A pharmaceutical composition according to any of claims 1-5 for oral or rectal administration.

7. A pharmaceutical composition according to any of claims 1-6 for the treatment of acute liver injury and failure and associated bacterial translocation in mammals including man.

8. A composition comprising a combination of a strain of Lactobacillus plantarum and arginine for use in therapy.

9. The use of a composition according to any of claims 1-6 and 8 in the manufacture of a medicament for the curative and/or prophylactic treatment of liver injury and failure and associated bacterial translocation in mammals including man.

## Patentansprüche

1. Pharmazeutische Zusammensetzung, umfassend
- einen Lactobacillus plantarum-Stamm mit der Fähigkeit, menschliche Darmschleimhaut in vivo zu besiedeln;
- Arginin; und
- einen pharmazeutisch annehmbaren Träger.

2. Pharmazeutische Zusammensetzung nach Anspruch 1, wobei der Lactobacillus plantarum-Stamm ein Adhäsin aufweist, das eine Anhaftung an einem Rezeptor auf menschlichen Darmepithelzellen ermöglicht.

3. Pharmazeutische Zusammensetzung nach Anspruch 1 oder 2, wobei der Lactobacillusstamm ein Stamm von Lactobacillus plantarum ist, der zu einem Cluster gehört, der eine Ähnlichkeit der Restriktionsendonukleasenanalyse von mehr als 70 % mit einem Stamm Lactobacillus plantarum 299, Hinterlegungsnummer DSM 6595, unter Verwendung des Pearson-Produkt-Moment-Korrelationskoeffizienten und des ungewichteten Paargruppenalgorithmus mit arithmetischen Mittelwerten (unweighed pair group algorithm with aritmethic averages) (UPGMA; GelCompare 3.0, Applied Maths, Kortrijk, Belgien) aufweist.

4. Pharmazeutische Zusammensetzung nach einem der Ansprüche 1-3, wobei der Lactobacillus plantarum-Stamm ausgewählt wird aus der Gruppe bestehend aus Lactobacillus plantarum 299, DSM 6595, und Lactobacillus plantarum 299v, DSM 9843.

5. Pharmazeutische Zusammensetzung nach einem der Ansprüche 1-4, wobei das Arginin L-Arginin ist.

6. Pharmazeutische Zusammensetzung nach einem der Ansprüche 1-5 für eine orale oder rektale Verabreichung.

7. Pharmazeutische Zusammensetzung nach einem der Ansprüche 1-6 für die Behandlung eines akuten Leberschadens und -versagens und einer damit verbundenen bakteriellen Translokation bei Säugern, einschließlich des Menschen.

8. Zusammensetzung, umfassend eine Kombination aus einem Stamm von Lactobacillus plantarum und Arginin, zur Verwendung in einer Therapie.

9. Verwendung einer Zusammensetzung nach einem der Ansprüche 1-6 und 8 bei der Herstellung eines Arzneimittels für die heilende und/oder prophylaktische Behandlung eines Leberschadens und -versagens und einer damit verbundenen bakteriellen Translokation bei Säugern, einschließlich des Menschen.

## Revendications

1. Composition pharmaceutique comprenant :
- une souche de Lactobacillus plantarum ayant l'aptitude à coloniser la muqueuse intestinale humaine *in vivo* ;
- de l'arginine ; et
- un véhicule pharmaceutiquement acceptable.

2. Composition pharmaceutique selon la revendication 1, dans laquelle la souche de Lactobacillus plantarum a une adhésine conférant de l'adhérence à un récepteur sur les cellules épithéliales de l'intestin humain.

3. Composition pharmaceutique selon la revendication 1 ou 2, dans laquelle la souche Lactobacillus est une souche de Lactobacillus plantarum appartenant à un amas ayant une similitude d'analyse d'endonucléases de restriction supérieure à 70 % par rapport à une souche de Lactobacillus plantarum 299, numéro de dépôt DSM 6595, en utilisant le coefficient de corrélation de Pearson et l'algorithme de groupes par paire non pondéré avec des moyennes arithmétiques (UPGMA ; GelCompare 3.0, Applied Maths, Kortrijk, Belgique).

4. Composition pharmaceutique selon l'une quelconque des revendications 1 à 3, dans laquelle la souche de Lactobacillus plantarum est choisie dans le groupe se composant de Lactobacillus plantarum 299, DSM 6595, et de Lactobacillus plantarum 299v, DSM 9843.

5. Composition pharmaceutique selon l'une quelconque des revendications 1 à 4, dans laquelle l'arginine est la L-arginine.

6. Composition pharmaceutique selon l'une quelconque des revendications 1 à 5, destinée à une administration orale ou rectale.

7. Composition pharmaceutique selon l'une quelconque des revendications 1 à 6, destinée au traitement de lésions et d'une insuffisance hépatiques aiguës et de la translocation bactérienne associée chez les mammifères, y compris chez l'homme.

8. Composition comprenant une combinaison d'une souche de Lactobacillus plantarum et d'arginine destinée à être utilisée dans une thérapie.

9. Utilisation d'une composition selon l'une quelconque des revendications 1 à 6 et 8 dans la fabrication d'un médicament pour délivrer un traitement curatif et/ou prophylactique pour des lésions et une insuffisance hépatiques et de la translocation bactérienne associée chez les mammifères, y compris chez l'homme.
